# EUROPEAN PATENT SPECIFICATION

(11) **EP 3 592 294 B1**
(45) Date of publication and mention of the grant of the patent: **18.12.2024**
(21) Application number: 18713464.8
(22) Date of filing: 06.03.2018
(51) Int. Cl.: A61F 2/24

(54) **REPLACEMENT HEART VALVE SYSTEM HAVING DOCKING STATION WITH SACRIFICIAL VALVE**
HERZKLAPPENERSATZSYSTEM MIT ANDOCKSTATION MIT OPFERVENTIL
SYSTÈME DE VALVULE CARDIAQUE DE REMPLACEMENT AYANT UNE STATION D'ANCRAGE AVEC VALVULE SACRIFICIELLE

(30) Priority: 06.03.2017 US 201762467599 P
(43) Date of publication of application: 15.01.2020
(73) Proprietor: Boston Scientific Scimed, Inc., Maple Grove, MN 55311 (US)
(72) Inventor: O'CONNOR, Tim, Claregalway Co.Galway (IE); MCNAMARA, Adrian, Galway (IE); O'GORMAN, Fionnuala, Dublin (IE); O'SULLIVAN, Richard, Turloughmore (IE); GUNNING, Paul, Roscommon (IE); QUIRKE, Kenneth, Galway (IE)
(74) Representative: Peterreins Schley
(86) International application number: PCT/US2018/021004
(87) International publication number: WO 2018/165064

(56) References cited:
- WO-A2-2012/178115
- US-A1- 2009 281 609
- US-A1- 2015 173 898

## Description

### Technical Field

The present disclosure pertains to medical devices, and methods for manufacturing and/or using medical devices. More particularly, the present disclosure pertains to a replacement heart valve system and/or apparatus and methods of manufacture therefor.

### Background

A wide variety of intracorporeal medical devices have been developed for medical use, for example, surgical and/or intravascular use. Some of these devices include guidewires, catheters, medical device delivery systems (e.g., for stents, grafts, replacement valves, etc.), and the like. These devices are manufactured by any one of a variety of different manufacturing methods and may be used according to any one of a variety of methods. Of the known medical devices and methods, each has certain advantages and disadvantages. There is an ongoing need to provide alternative medical devices as well as alternative methods for manufacturing and/or using medical devices.

US 2009/281609 A1 discloses a two-step heart valve implantation using a two-part heart valve implant.

### Summary

The present invention is directed to a replacement heart valve system as set forth in the claims. According to the invention, the replacement heart valve system comprises an expandable docking station configured for implantation within a native heart valve, the expandable docking station including a plurality of sacrificial valve leaflets disposed within an anchoring element defining a lumen extending through the anchoring element; and a replacement heart valve implant configured to be disposed within the lumen of the expandable docking station, the replacement heart valve implant including a plurality of valve leaflets disposed within a tubular anchor member defining a lumen extending through the tubular anchor member.

In addition or alternatively, and in a second aspect, the plurality of sacrificial valve leaflets is configured to move between an open configuration permitting antegrade fluid flow through the expandable docking station and a closed configuration preventing retrograde fluid flow through the expandable docking station.

In addition or alternatively, and in a third aspect, the plurality of valve leaflets is configured to move between an open configuration permitting antegrade fluid flow through the replacement heart valve implant and a closed configuration preventing retrograde fluid flow through the replacement heart valve implant.

In addition or alternatively, and in a fourth aspect, the anchoring element is configured to shift between an elongated delivery configuration and a shortened, radially expanded deployed configuration.

In addition or alternatively, and in a fifth aspect, the anchoring element is configured to clamp native valve leaflets of the native heart valve in the deployed configuration.

In addition or alternatively, and in a sixth aspect, the anchoring element comprises an upstream flange, a downstream flange, and a central portion disposed between the upstream flange and the downstream flange.

In addition or alternatively, and in a seventh aspect, the upstream flange extends radially outward of the central portion in the deployed configuration.

In addition or alternatively, and in an eighth aspect, the downstream flange extends radially outward of the central portion in the deployed configuration.

In addition or alternatively, and in a ninth aspect, the expandable docking station includes a seal member disposed on at least a portion of an outer surface of the anchoring element.

In addition or alternatively, and in a tenth aspect, the seal member is disposed on the central portion of the anchoring element.

In addition or alternatively, and in an eleventh aspect, the tubular anchor member is configured to shift between an elongated delivery configuration and a shortened, radially expanded deployed configuration.

In addition or alternatively, and in a twelfth aspect, the tubular anchor member is configured to engage the anchoring element in the deployed configuration of the tubular anchor member.

In addition or alternatively, and in a thirteenth aspect, when the tubular anchor member is in the deployed configuration within the anchoring element, the plurality of sacrificial valve leaflets is compressed between the tubular anchor member and the anchoring element thereby forming a fluid tight seal therebetween.

In addition or alternatively, and in a fourteenth aspect, the expandable docking station includes a leaflet subframe disposed within the lumen of the anchoring element.

In addition or alternatively, and in a fifteenth aspect, a replacement heart valve system may comprise an expandable docking station configured for implantation within a native heart valve, the expandable docking station including a plurality of sacrificial valve leaflets disposed within a braided anchoring element defining a lumen extending through the braided anchoring element; a replacement heart valve implant configured to be disposed within the lumen of the expandable docking station, the replacement heart valve implant including a plurality of valve leaflets disposed within a tubular anchor member defining a lumen extending through the tubular anchor member; a docking station delivery device, wherein the expandable docking station is disposed within a lumen of the docking station delivery device in the delivery configuration for delivery to the native heart valve; and a replacement heart valve delivery device, wherein the replacement heart valve implant is disposed within a lumen of the replacement heart valve delivery device in the delivery configuration for delivery to the expandable docking station disposed within the native heart valve.

Also disclosed herein is a method of deploying a replacement heart valve implant within a native heart valve that may comprise advancing a docking station delivery device having an expandable docking station disposed within a lumen of the docking station delivery device in a delivery configuration to the native heart valve, wherein the expandable docking station includes a plurality of sacrificial valve leaflets disposed within an anchoring element defining a lumen extending through the anchoring element; deploying the expandable docking station from the docking station delivery device into the native heart valve and expanding the expandable docking station to a deployed configuration; and deploying a replacement heart valve implant within the lumen of the anchoring element, the replacement heart valve implant including a plurality of valve leaflets disposed within a tubular anchor member defining a lumen extending through the tubular anchor member.

In addition or alternatively, the method may further comprise advancing a replacement heart valve delivery device having the replacement heart valve implant disposed within a lumen of the replacement heart valve delivery device in a delivery configuration to the native heart valve, wherein deploying the replacement heart valve implant includes deploying the replacement heart valve implant from the replacement heart valve delivery device into the lumen of the anchoring element and expanding the replacement heart valve implant to a deployed configuration.

In addition or alternatively, in the deployed configuration, the tubular anchor member of the replacement heart valve implant is engaged with the anchoring element of the expandable docking station.

In addition or alternatively, deploying the replacement heart valve implant within the lumen of the anchoring element compresses the plurality of sacrificial valve leaflets between the tubular anchor member and the anchoring element thereby forming a fluid tight seal therebetween.

In addition or alternatively, the expandable docking station includes a seal member disposed on at least a portion of an outer surface of the anchoring element.

The above summary of some embodiments, aspects, and/or examples is not intended to describe each embodiment or every implementation of the present disclosure. The figures and the detailed description which follows more particularly exemplify these embodiments.

### Brief Description of the Drawings

The disclosure may be more completely understood in consideration of the following detailed description of various embodiments in connection with the accompanying drawings, in which:
FIG. 1 is a partial cut-away view of an example heart;
FIG. 2 illustrates several possible approaches for delivery of a replacement heart valve;
FIG. 3 illustrates an example docking station delivery device of an example replacement heart valve system with an example docking station in a delivery configuration;
FIG. 4 illustrates the example docking station of FIG. 3 in a delivery configuration;
FIG. 5 illustrates the docking station of FIGS. 3-4 in a deployed configuration;
FIG. 6 illustrates the docking station of FIG. 5 deployed in a native heart valve; and
FIG. 7 is a partial cross-sectional view of the docking station of FIGS. 3-5 in the deployed configuration;
FIG. 8 illustrates an example docking station according to an alternative embodiment of the disclosure;
FIG. 9 illustrates an example replacement heart valve delivery device of an example replacement heart valve system;
FIG. 10 illustrates the example replacement heart valve delivery device of FIG. 9 with a replacement heart valve implant partially deployed within an example docking station; and
FIG. 11 illustrates an example replacement heart valve implant deployed within an example docking station.

While aspects of the disclosure are amenable to various modifications and alternative forms, specifics thereof have been shown by way of example in the drawings and will be described in detail. It should be understood, however, that the intention is not to limit aspects of the disclosure to the particular embodiments described.

### Detailed Description

The following description should be read with reference to the drawings, which are not necessarily to scale, wherein like reference numerals indicate like elements throughout the several views. The detailed description and drawings are intended to illustrate but not limit the claimed invention. Those skilled in the art will recognize that the various elements described and/or shown may be arranged in various combinations and configurations without departing from the scope of the disclosure. The detailed description and drawings illustrate example embodiments of the claimed invention.

For the following defined terms, these definitions shall be applied, unless a different definition is given in the claims or elsewhere in this specification.

All numeric values are herein assumed to be modified by the term "about," whether or not explicitly indicated. The term "about", in the context of numeric values, generally refers to a range of numbers that one of skill in the art would consider equivalent to the recited value (e.g., having the same function or result). In many instances, the term "about" may include numbers that are rounded to the nearest significant figure. Other uses of the term "about" (e.g., in a context other than numeric values) may be assumed to have their ordinary and customary definition(s), as understood from and consistent with the context of the specification, unless otherwise specified.

The recitation of numerical ranges by endpoints includes all numbers within that range, including the endpoints (e.g., 1 to 5 includes 1, 1.5, 2, 2.75, 3, 3.80, 4, and 5).

Although some suitable dimensions, ranges, and/or values pertaining to various components, features and/or specifications are disclosed, one of skill in the art, incited by the present disclosure, would understand desired dimensions, ranges, and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise. It is to be noted that in order to facilitate understanding, certain features of the disclosure may be described in the singular, even though those features may be plural or recurring within the disclosed embodiment(s). Each instance of the features may include and/or be encompassed by the singular disclosure(s), unless expressly stated to the contrary. For simplicity and clarity purposes, not all elements of the disclosed invention are necessarily shown in each figure or discussed in detail below. However, it will be understood that the following discussion may apply equally to any and/or all of the components for which there are more than one, unless explicitly stated to the contrary. Additionally, not all instances of some elements or features may be shown in each figure for clarity.

Relative terms such as "proximal", "distal", "advance", "retract", variants thereof, and the like, may be generally considered with respect to the positioning, direction, and/or operation of various elements relative to a user/operator/manipulator of the device, wherein "proximal" and "retract" indicate or refer to closer to or toward the user and "distal" and "advance" indicate or refer to farther from or away from the user. In some instances, the terms "proximal" and "distal" may be arbitrarily assigned in an effort to facilitate understanding of the disclosure, and such instances will be readily apparent to the skilled artisan. Other relative terms, such as "upstream", "downstream", "inflow", and "outflow" refer to a direction of fluid flow within a lumen, such as a body lumen, a blood vessel, or within a device.

The term "extent" may be understood to mean a greatest measurement of a stated or identified dimension. For example, "outer extent" may be understood to mean a maximum outer dimension, "radial extent" may be understood to mean a maximum radial dimension, "longitudinal extent" may be understood to mean a maximum longitudinal dimension, etc. Each instance of an "extent" may be different (e.g., axial, longitudinal, lateral, radial, circumferential, etc.) and will be apparent to the skilled person from the context of the individual usage. Generally, an "extent" may be considered a greatest possible dimension measured according to the intended usage. In some instances, an "extent" may generally be measured orthogonally within a plane and/or cross-section, but may be, as will be apparent from the particular context, measured differently - such as, but not limited to, angularly, radially, circumferentially (e.g., along an arc), etc.

It is noted that references in the specification to "an embodiment", "some embodiments", "other embodiments", etc., indicate that the embodiment(s) described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it would be within the knowledge of one skilled in the art to effect the particular feature, structure, or characteristic in connection with other embodiments, whether or not explicitly described, unless clearly stated to the contrary. That is, the various individual elements described below, even if not explicitly shown in a particular combination, are nevertheless contemplated as being combinable or arrangeable with each other to form other additional embodiments or to complement and/or enrich the described embodiment(s), as would be understood by one of ordinary skill in the art.

For the purpose of clarity, certain identifying numerical nomenclature (e.g., first, second, third, fourth, etc.) may be used throughout the description and/or claims to name and/or differentiate between various described and/or claimed features. It is to be understood that the numerical nomenclature is not intended to be limiting and is exemplary only. In some embodiments, alterations of and deviations from previously-used numerical nomenclature may be made in the interest of brevity and clarity. That is, a feature identified as a "first" element may later be referred to as a "second" element, a "third" element, etc. or may be omitted entirely, and/or a different feature may be referred to as the "first" element. The meaning and/or designation in each instance will be apparent to the skilled practitioner.

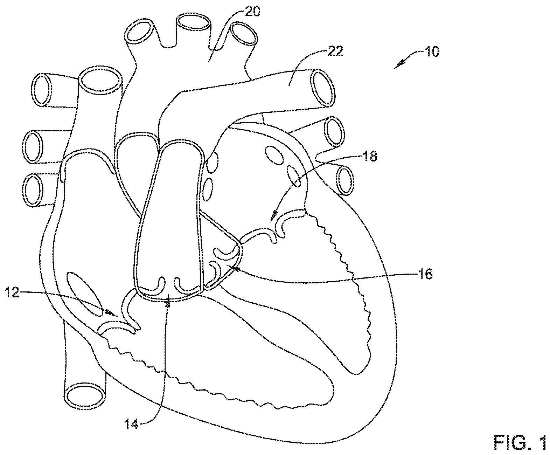

Diseases and/or medical conditions that impact the cardiovascular system are prevalent throughout the world. Some mammalian hearts (e.g., human, etc.) include four heart valves: a tricuspid valve 12, a pulmonary valve 14, an aortic valve 16, and a mitral valve 18, as seen in an example heart 10 illustrated in FIG. 1. The purpose of the heart valves is to allow blood to flow through the heart 10 and from the heart 10 into the major blood vessels connected to the heart 10, such as the aorta 20 and the pulmonary artery 22, for example. In a normally functioning heart valve, blood is permitted to pass or flow downstream through the heart valve (e.g., from an atrium to a ventricle, from a ventricle to an artery, etc.) when the heart valve is open, and when the heart valve is closed, blood is prevented from passing or flowing back upstream through the heart valve (e.g., from a ventricle to an atrium, etc.). Some relatively common medical conditions may include or be the result of inefficiency, ineffectiveness, or complete failure of one or more of the valves within the heart. Treatment of defective heart valves poses other challenges in that the treatment often requires the repair or outright replacement of the defective valve. Such therapies may be highly invasive to the patient. Disclosed herein are medical devices that may be used within a portion of the cardiovascular system in order to diagnose, treat, and/or repair the system. At least some of the medical devices disclosed herein may include a replacement heart valve (e.g., a replacement aortic valve, a replacement mitral valve, etc.) and may reduce, treat, and/or prevent the occurrence of defects such as (but not limited to) regurgitation, leaflet prolapse, and/or valve stenosis. In addition, the devices disclosed herein may deliver the replacement heart valve percutaneously and, thus, may be much less invasive to the patient, although other surgical methods and approaches may also be used. The devices disclosed herein may also provide a number of additional desirable features and benefits as described in more detail below. For the purpose of this disclosure, the discussion below is directed toward a replacement mitral valve and will be so described in the interest of brevity. This, however, is not intended to be limiting as the skilled person will recognize that the following discussion may also apply to a replacement aortic valve or another replacement heart valve with no or minimal changes to the structure and/or scope of the disclosure.

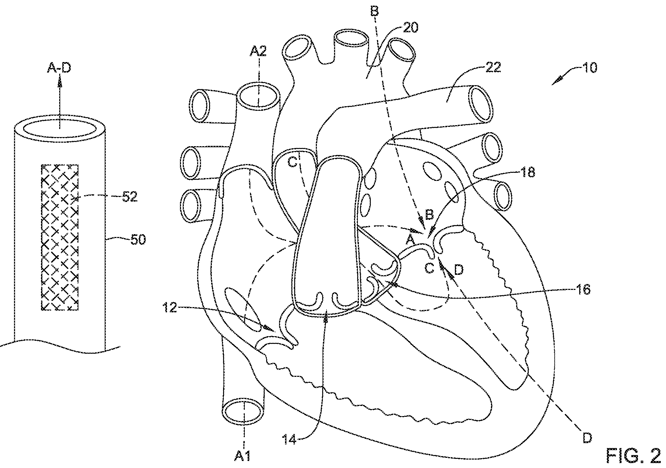

An example replacement heart valve system may include a delivery sheath 50 (or multiple delivery sheaths 50), such as those known and/or used with replacement heart valves, and one or more implants and/or medical devices 52 configured for implantation within an annulus of a native heart valve. For the purpose of this disclosure, reference numerals 50 and 52 may be considered generic placeholders that may be interchanged with other corresponding reference numerals used in the embodiment(s) described below. The specific corresponding features will be readily apparent to the skilled person. The replacement heart valve system may include the implants and/or medical devices 52 disposed within a lumen of the delivery sheath 50 proximate a distal end of the delivery sheath 50 in an elongated delivery configuration, as seen in FIG. 2 for example, the implants and/or medical devices 52 being expandable to a deployed configuration when unconstrained by the delivery sheath 50. Further details regarding the replacement heart valve system, the delivery sheath(s) 50, and/or the implants and/or medical devices 52 will be described below. FIG. 2 illustrates several possible approaches that may be used to deliver the replacement heart valve system and/or the implants and/or medical devices 52 to the mitral valve 18 with the delivery sheath(s) 50.

In some embodiments, the implants and/or medical devices 52 may be delivered percutaneously to the mitral valve 18 via a transseptal approach "A". Within the transseptal approach "A", which involves transiting the septum of the heart 10, the delivery sheath 50 may be advanced into right atrium of the heart 10 through the inferior vena cava ("A1") or the superior vena cava ("A2") before transiting the septum. In some embodiments, the implants and/or medical devices 52 may be delivered surgically to the mitral valve 18 via a left atriotomy "B", which involves surgically opening the left atrium of the heart 10. In some embodiments, the implants and/or medical devices 52 may be delivered percutaneously to the mitral valve 18 via a transaortic approach "C", which involves transiting the aorta 20 (from a femoral entry point in some cases), the aortic arch, the aortic valve 16, and the left ventricle of the heart 10. In some embodiments, the implants and/or medical devices 52 may be delivered surgically to the mitral valve 18 via a transapical approach "D", which involves transiting the wall of the left ventricle of the heart 10. Certain constructional details of the delivery sheath(s) 50 (e.g., length, stiffness, column strength, etc.) may be adjusted according to the approach used in any given procedure. Orientation of the implants and/or medical devices 52 within the delivery sheath(s) 50 may be adjusted depending upon the selected approach used in any given procedure. Some suitable but non-limiting materials for the delivery sheath(s) 50, for example metallic materials, polymer materials, composite materials, etc., are described below.

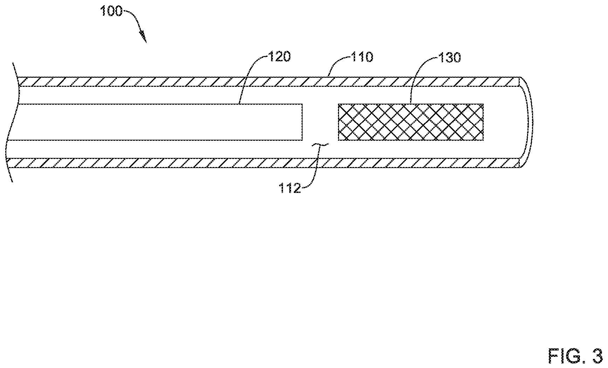

FIG. 3 illustrates a docking station delivery device 100 including an expandable docking station 130 configured for implantation within a native heart valve (e.g., mitral valve 18, aortic valve 16, etc.), the expandable docking station 130 disposed within a lumen of the docking station delivery device 100 in a delivery configuration. In some embodiments, the docking station delivery device 100 may include a delivery sheath 110 having a lumen 112 extending from a proximal portion and/or proximal end of the delivery sheath 110 to a distal end of the delivery sheath 110. In some embodiments, the docking station delivery device 100 may include a handle (not shown) disposed proximate the proximal end of the delivery sheath 110. In one example, the expandable docking station 130 may be considered to correspond to an implant and/or medical device 52 above, and the delivery sheath 110 may be considered to correspond to a delivery sheath 50. For the purpose of the disclosure and ease of understanding, the terms may be used interchangeably herein.

The docking station delivery device 100 may include an elongate shaft 120 disposed within the lumen 112 of the delivery sheath 110 and/or slidable with respect to the delivery sheath 110 within the lumen 112 of the delivery sheath 110. In some embodiments, the elongate shaft 120 may be a tubular structure having a lumen extending therethrough, the elongate shaft 120 may be a solid shaft, or the elongate shaft 120 may be a combination thereof. In use, the elongate shaft 120 may be used to move the expandable docking station 130 with respect to the delivery sheath 110 of the docking station delivery device 100. For example, the elongate shaft 120 may be advanced distally within the lumen 112 of the delivery sheath 110 to push the expandable docking station 130 out the distal end of the delivery sheath 110 and/or the docking station delivery device 100 to deploy the expandable docking station 130 in the native heart valve. Alternatively, the elongate shaft 120 may be held in a fixed position relative to the expandable docking station 130 and the delivery sheath 110 may be withdrawn proximally relative to the elongate shaft 120 and/or the expandable docking station 130 to deploy the expandable docking station 130 in the native heart valve. Some examples of suitable but non-limiting materials for the docking station delivery device 100, the delivery sheath 110, the elongate shaft 120, and/or components or elements thereof, are described below.

In some embodiments, the expandable docking station 130 may include one or more suture loops attached thereto. In embodiments having more than one suture loop, the suture loops may be spaced apart around a circumference of the expandable docking station 130. The one or more suture loops may be used in delivering the expandable docking station 130 to the native heart valve, and may extend through the delivery sheath 110 of the docking station delivery device 100 and/or may be attached to the elongate shaft 120 of the docking station delivery device 100. The one or more suture loops may permit retrieval of the expandable docking station 130 during a deployment procedure, for example, if the expandable docking station 130 needs to be repositioned. In some embodiments, after deploying the expandable docking station 130 in the native heart valve, the one or more suture loops may remain attached thereto, thus permitting later retrieval of the expandable docking station 130.

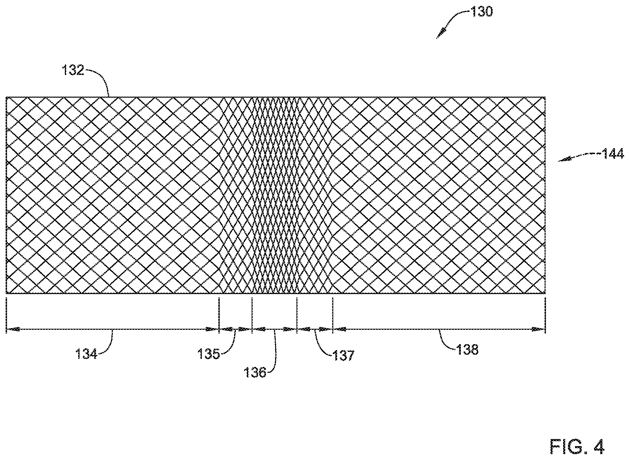

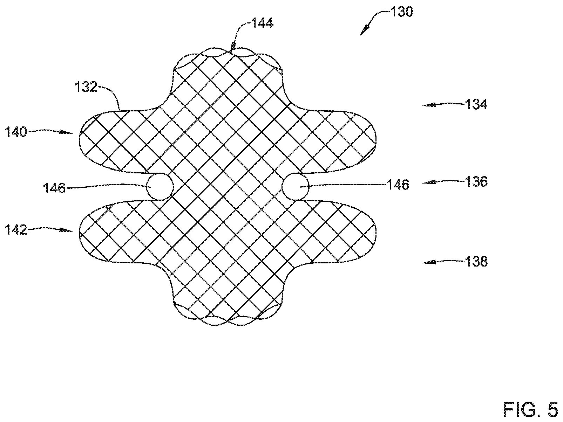

FIG. 4 illustrates some additional details of the expandable docking station 130, shown in an elongated delivery configuration. According to the invention, the expandable docking station 130 includes a braided anchoring element 132 defining a lumen 144 extending through the braided anchoring element 132. In some embodiments, the expandable docking station 130 and/or the braided anchoring element 132 may include a first end portion 134, a central portion 136, and a second end portion 138 disposed opposite the first end portion 134 relative to the central portion 136 and/or with the central portion 136 disposed between the first end portion 134 and the second end portion 138. In at least some embodiments, the braided anchoring element 132 may comprise a self-expanding braided and/or woven mesh structure made up of one or more filaments disposed and/or interwoven circumferentially about the lumen 144 of the braided anchoring element 132 and/or the expandable docking station 130. Non-self-expanding, mechanically-expandable, and/or assisted self-expanding braided anchoring elements are also contemplated. In at least some embodiments, the braided anchoring element 132 may be formed as a unitary structure (e.g., formed from a single filament or strand of wire, cut from a single tubular member, etc.). The first end portion 134 may form an upstream flange 140 in a shortened, radially expanded deployed configuration, the second end portion 138 may form a downstream flange 142 in the shortened, radially expanded deployed configuration (see FIG. 5 for example), and the central portion 136 may be disposed between the upstream flange 140 and the downstream flange 142.

In some embodiments, the first end portion 134 and/or the second end portion 138 may have a relatively large pitch angle and/or spacing between adjacent windings of the one or more filaments. In one example, the first end portion 134 and/or the second end portion 138 may have a pitch angle of about 50 degrees. Other suitable pitch angles, including but not limited to, 35 degrees, 45 degrees, 60 degrees, etc. are also contemplated. The pitch angle and/or spacing between adjacent windings of the one or more filaments of the first end portion 134 and/or the second end portion 138 may provide a relatively low radially outward force allowing the first end portion 134 and/or the upstream flange 140, and/or the second end portion 138 and/or the downstream flange 142, to substantially conform to the native heart valve's anatomy, thereby aiding in sealing and mitigating leakage around the periphery of the expandable docking station 130 and/or the braided anchoring element 132.

In some embodiments, the central portion 136 may have a relatively low pitch angle and/or spacing between adjacent windings of the one or more filaments. In one example, the central portion 136 may have a pitch angle of about 10 degrees. Other suitable pitch angles, including but not limited to, 5 degrees, 15 degrees, etc. are also contemplated. The pitch angle and/or spacing between adjacent windings of the one or more filaments of the central portion 136 may provide a relatively high radially outward force designed to form a substantially circular orifice configured to receive and/or accept a replacement heart valve implant (discussed below). The relatively high radially outward force may overcome the natural stiffness of the native tissue/anatomy and remodel the native valve opening into the substantially circular orifice to provide a consistent receptacle for seating the replacement heart valve implant, thereby potentially improving replacement heart valve implant leaflet function and durability.

In some embodiments, the braided anchoring element 132 may include a first transition zone 135 between the central portion 136 and the first end portion 134 having a relative pitch angle and/or spacing between adjacent windings of the one or more filaments intermediate to the pitch angle of the first end portion 134 and the pitch angle of the central portion 136. For example, the first transition zone 135 may have a pitch angle of about 20 degrees, about 25 degrees, about 30 degrees, etc. Similarly, in some embodiments, the braided anchoring element 132 may include a second transition zone 137 between the central portion 136 and the second end portion 138 having a relative pitch angle and/or spacing between adjacent windings of the one or more filaments intermediate to the pitch angle of the second end portion 138 and the pitch angle of the central portion 136. For example, the second transition zone 137 may have a pitch angle of about 20 degrees, about 25 degrees, about 30 degrees, etc.

In some embodiments, the varying pitch angle and/or spacing between adjacent windings of the one or more filaments along the axial and/or longitudinal length of the braided anchoring element 132 may permit the braided anchoring element 132 and/or the expandable docking station 130 to deploy out of the delivery sheath 110 and/or the docking station delivery device 100 with a varying speed and/or degree of spring rate. For example, the first end portion 134 and/or the second end portion 138, having a relatively large pitch angle and/or spacing between adjacent windings of the one or more filaments, may form, recoil, and/or expand to the deployed configuration (shown in FIG. 5 for example) relatively slowly (e.g., with a low spring rate), while the central portion 136, having a relatively low pitch angle and/or spacing between adjacent windings of the one or more filaments, may form, recoil, and/or expand to the deployed configuration relatively quickly (e.g., with a high spring rate). In some embodiments, the variable speed at which the braided anchoring element 132 and/or the expandable docking station 130 may be deployed allows for fast and more effective encapsulation, capture, and/or clamping of the native heart valve leaflets.

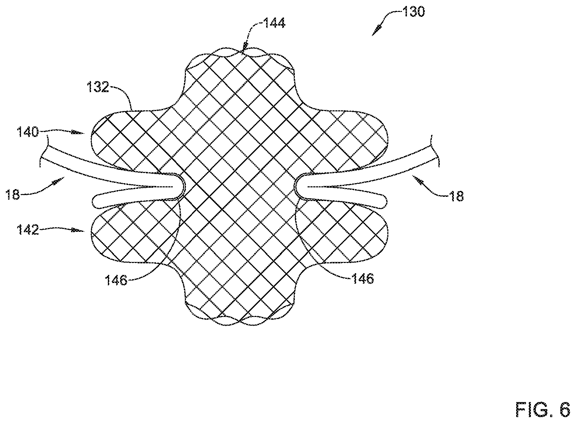

The expandable docking station 130 and/or the braided anchoring element 132 may be configured to shift between the elongated delivery configuration and the shortened, radially expanded deployed configuration. FIG. 5 illustrates the expandable docking station 130 and/or the braided anchoring element 132 in the shortened, radially expanded deployed configuration. As mentioned above, the first end portion 134 may form an upstream flange 140, the second end portion 138 may form a downstream flange 142 and the central portion 136 may be disposed therebetween. An interior-facing surface of the braided anchoring element 132 may define the lumen 144 extending through the braided anchoring element 132. In some embodiments, the upstream flange 140 may extend radially outward of the central portion 136 in the deployed configuration. In some embodiments, the downstream flange 142 may extend radially outward of the central portion 136 in the deployed configuration. In some embodiments, the braided anchoring element 132 may be configured to clamp native valve leaflets of the native heart valve between the upstream flange 140 and the downstream flange 142 in the deployed configuration, as seen in FIG. 6 for example.

In some embodiments, the expandable docking station 130 and/or the braided anchoring element 132 may include one or more barbs configured to attach the braided anchoring element 132 to the native heart valve and/or native valve leaflets. In some embodiments, the one or more barbs may extend from the upstream flange 140 and/or the downstream flange 142 in the deployed configuration. In some embodiments, the one or more barbs may extend from a radially outermost edge and/or extent of the upstream flange 140 and/or the downstream flange 142 in the deployed configuration. The one or more barbs may assist in preventing migration of the expandable docking station 130 and/or the braided anchoring element 132 relative to the native heart valve. Some examples of suitable but non-limiting materials for the barbs, the braided anchoring element 132, and/or components or elements thereof, are described below.

In some embodiments, the expandable docking station 130 may include a seal member 146 disposed on and/or about at least a portion of an outer surface of the braided anchoring element 132. In at least some embodiments, the seal member 146 may be disposed on and/or about the central portion 136 of the braided anchoring element 132. In some embodiments, the seal member 146 may be coupled and/or secured to the braided anchoring element 132. As seen in FIG. 6 for example, the seal member 146 may be sufficiently flexible and/or pliable to conform to and/or around native valve leaflets and/or the native heart valve in the deployed configuration, thereby sealing an exterior of the expandable docking station 130 within and/or against the native heart valve and/or the native valve leaflets and preventing leakage around the expandable docking station 130 and/or the braided anchoring element 132.

In some embodiments, the seal member 146 may include a plurality of layers of polymeric material. Some suitable polymeric materials may include, but are not necessarily limited to, polycarbonate, polyurethane, polyamide, polyether block amide, polyethylene, polyethylene terephthalate, polypropylene, polyvinylchloride, polytetrafluoroethylene, polysulfone, and copolymers, blends, mixtures or combinations thereof. Other suitable polymeric materials are also contemplated, some of which are discussed below.

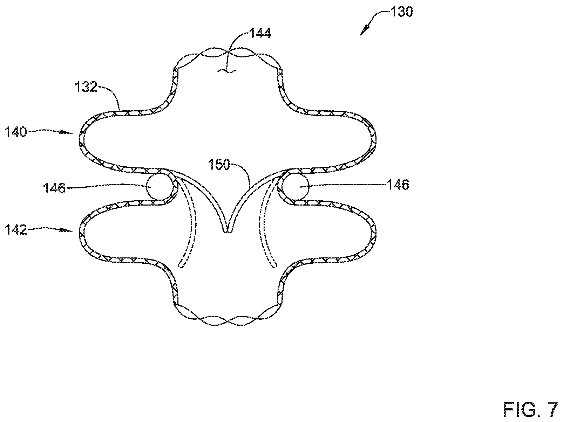

FIG. 7 illustrates the expandable docking station 130 in partial cross-section. As shown, the expandable docking station 130 includes a plurality of sacrificial valve leaflets 150 disposed within the braided anchoring element 132 and/or the lumen 144 extending through the braided anchoring element 132. In some embodiments, the plurality of sacrificial valve leaflets 150 may be attached and/or coupled to the braided anchoring element 132. In some embodiments, the plurality of sacrificial valve leaflets 150 may be attached and/or coupled to the braided anchoring element 132 using sutures, adhesives, or other suitable means. The plurality of sacrificial valve leaflets 150 may comprise two valve leaflets, three valve leaflets, four valve leaflets, or another suitable number of valve leaflets as desired. In some embodiments, the plurality of valve leaflets 150 may be configured to move between an open configuration (shown in phantom in FIG. 7) permitting antegrade fluid flow through the expandable docking station 130, the braided anchoring element 132, and/or the lumen 144 extending through the braided anchoring element 132, and a closed configuration preventing retrograde fluid flow through the expandable docking station 130, the braided anchoring element 132, and/or the lumen 144 extending through the braided anchoring element 132. The plurality of sacrificial valve leaflets 150 may each have a free edge, wherein the free edges of the plurality of sacrificial valve leaflets 150 coapt within the expandable docking station 130, the braided anchoring element 132, and/or the lumen 144 extending through the braided anchoring element 132 in the closed configuration. The plurality of sacrificial valve leaflets 150 may provide temporary heart valve function during a period of time between deployment of the expandable docking station 130 and deployment of a replacement heart valve implant therein, as will be described below. The plurality of sacrificial valve leaflets 150 may prevent regurgitation through the lumen 144 extending through the braided anchoring element 132. Some examples of suitable but non-limiting materials for the plurality of sacrificial valve leaflets 150 are described below.

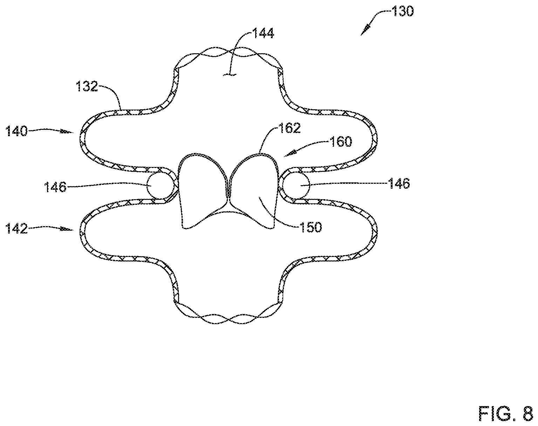

In some embodiments, the expandable docking station 130 may comprise the braided anchoring element 132 as an outer frame, and a leaflet subframe 160 having the plurality of sacrificial valve leaflets 150 attached thereto, the leaflet subframe 160 being disposed within the expandable docking station 130, the braided anchoring element 132, and/or the lumen 144 extending through the braided anchoring element 132, as shown in FIG. 8 for example. The leaflet subframe 160 may include a wire 162 formed into a plurality of commissures corresponding to the plurality of sacrificial valve leaflets 150. For example, for each sacrificial valve leaflet there may be one commissure formed in the leaflet subframe 160. Each of the plurality of sacrificial valve leaflets 150 may be attached to two adjacent commissures of the leaflet subframe 160, resulting in two adjacent sacrificial valve leaflets 150 each being attached to the same commissure and/or each other at the same commissure. The plurality of sacrificial valve leaflets 150 may each have a free edge, wherein the free edges of the plurality of sacrificial valve leaflets 150 coapt within the leaflet subframe 160 in the closed configuration. The leaflet subframe 160 may be attached to the braided anchoring element 132 at a plurality of locations, using sutures for example. In some embodiments, the leaflet subframe 160 may be attached to the braided anchoring element 132 along the wire 162 between adjacent commissures. In one non-limiting example, the leaflet subframe 160 may be attached to the braided anchoring element 132 at three discrete locations, approximately 120 degrees apart for example, thereby permitting relative movement between the braided anchoring element 132 and the leaflet subframe 160 for easily collapsing the expandable docking station 130 to the elongated delivery configuration. Other orientations and/or configurations are also contemplated, including but not limited to attachment at more or less than three discrete locations and/or varied spacing of the discrete locations. Some examples of suitable but non-limiting materials for the leaflet subframe 160 are described below.

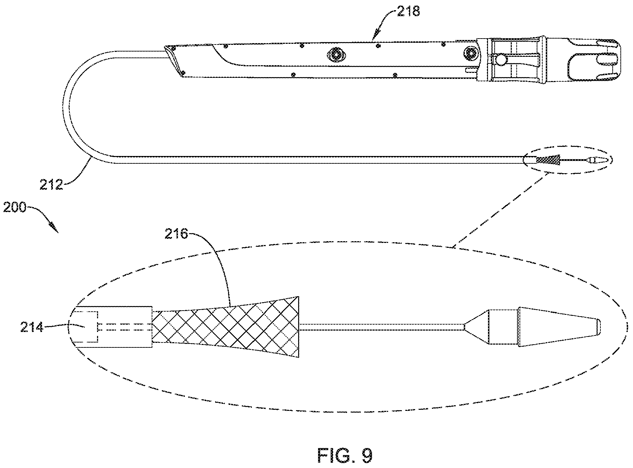

FIG. 9 illustrates a replacement heart valve delivery device 200 including a replacement heart valve implant 216 configured to be disposed within the lumen 144 of the expandable docking station 130 within a native heart valve (e.g., mitral valve 18, aortic valve 16, etc.), wherein the replacement heart valve implant 216 is disposed within a lumen of the replacement heart valve delivery device 200 in a delivery configuration for delivery to the lumen 144 extending through the braided anchoring element 132 of the expandable docking station 130 disposed within the native heart valve in the deployed configuration. In some embodiments, the replacement heart valve delivery device 200 may include a delivery sheath 212 having a lumen extending from a proximal portion and/or proximal end of the delivery sheath 212 to a distal end of the delivery sheath 212. In some embodiments, the replacement heart valve delivery device 200 may include a handle 218 disposed proximate the proximal end of the delivery sheath 212. In one example, the replacement heart valve implant 216 may be considered to correspond to an implant and/or medical device 52 above, and the delivery sheath 212 may be considered to correspond to a delivery sheath 50. For the purpose of the disclosure and ease of understanding, the terms may be used interchangeably herein.

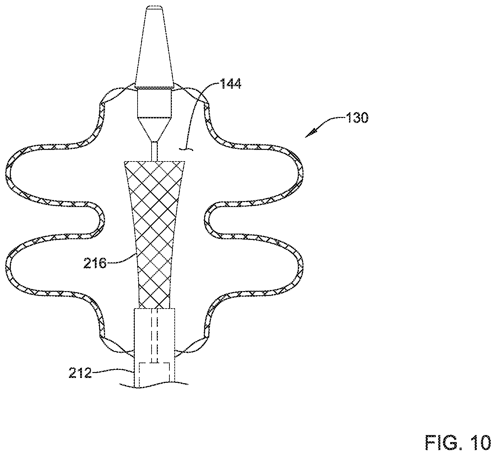

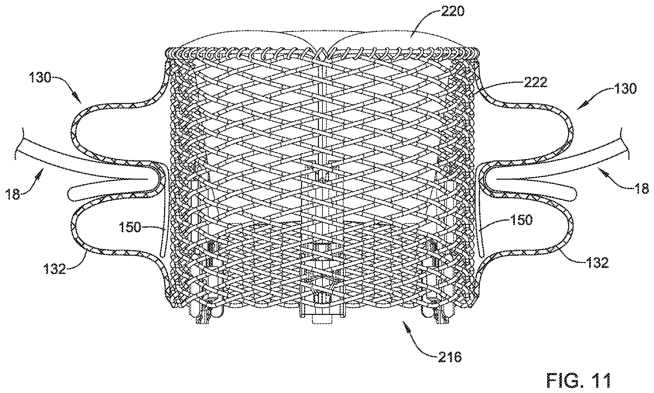

The replacement heart valve delivery device 200 may include an elongate shaft 214 disposed within the lumen of the delivery sheath 212 and/or slidable with respect to the delivery sheath 212 within the lumen of the delivery sheath 212. In some embodiments, the elongate shaft 214 may be a tubular structure having a lumen extending therethrough, the elongate shaft 214 may be a solid shaft, or the elongate shaft 214 may be a combination thereof. In use, the elongate shaft 214 may be used to move the replacement heart valve implant 216 with respect to the delivery sheath 212 of the replacement heart valve delivery device 200. For example, the elongate shaft 214 may be advanced distally within the lumen of the delivery sheath 212 to push the replacement heart valve implant 216 out the distal end of the delivery sheath 212 and/or the replacement heart valve delivery device 200 to deploy the replacement heart valve implant 216 within the lumen 144 of the expandable docking station 130 in the native heart valve. Alternatively, the elongate shaft 214 may be held in a fixed position relative to the replacement heart valve implant 216 and the delivery sheath 212 may be withdrawn proximally relative to the elongate shaft 214 and/or the replacement heart valve implant 216 to deploy the replacement heart valve implant 216 within the lumen 144 of the expandable docking station 130 in the native heart valve. Deployment of the replacement heart valve implant 216 into and/or within the lumen 144 of the expandable docking station 130 may be seen in FIGS. 10 and 11. Some examples of suitable but non-limiting materials for the replacement heart valve delivery device 200, the delivery sheath 212, the elongate shaft 214, and/or components or elements thereof, are described below.

Some additional details of an example replacement heart valve implant 216 may be seen in FIG. 11. According to the invention, the replacement heart valve implant 216 includes a plurality of valve leaflets 220 disposed within a tubular anchor member 222 defining a lumen extending through the tubular anchor member 222. The plurality of valve leaflets 220 may be attached and/or coupled to the tubular anchor member 222 at a plurality of locations. In some embodiments, the plurality of valve leaflets 220 may be attached and/or coupled to the tubular anchor member 222 using sutures, adhesives, or other suitable means. In some embodiments, the plurality of valve leaflets 220 may include two leaflets, three leaflets, four leaflets, etc. as desired. The plurality of valve leaflets 220 may each have a free edge, wherein the free edges of the plurality of valve leaflets 220 coapt within the replacement heart valve implant 216, the tubular anchor member 222, and/or the lumen extending through the tubular anchor member 222 in the closed configuration. The plurality of valve leaflets 220 of the replacement heart valve implant 216 may be configured to move between an open configuration permitting antegrade fluid flow through the replacement heart valve implant 216 and/or the lumen of the tubular anchor member 222 and a closed configuration preventing retrograde fluid flow through the replacement heart valve implant 216 and/or the lumen of the tubular anchor member 222.

The replacement heart valve implant 216 and/or the tubular anchor member 222 may be configured to shift between an elongated delivery configuration (e.g., FIGS. 9 and 10) and a shortened, radially expanded deployed configuration (e.g., FIG. 11). In some embodiments, the tubular anchor member 222 may comprise a self-expanding braided and/or woven mesh structure made up of one or more filaments disposed and/or interwoven circumferentially about the lumen of the tubular anchor member 222 and/or the replacement heart valve implant 216. Non-self-expanding, mechanically-expandable, and/or assisted self-expanding tubular anchor members are also contemplated. In at least some embodiments, the tubular anchor member 222 may be formed as a unitary structure (e.g., formed from a single filament or strand of wire, cut from a single tubular member, etc.). Some examples of suitable but non-limiting materials for replacement heart valve implant 216, the tubular anchor member 222, and/or components or elements thereof, are described below.

An outer surface of the tubular anchor member 222 may be configured to engage an interior-facing surface of the braided anchoring element 132 in the deployed configuration of the tubular anchor member 222. When the tubular anchor member 222 is in the deployed configuration within the braided anchoring element 132 of the expandable docking station 130, the plurality of sacrificial valve leaflets 150 may be compressed, pinched, squeezed, etc. between at least a portion of the outer surface of the tubular anchor member 222 and at least a portion of the interior-facing surface of the braided anchoring element 132, thereby forming a fluid tight seal therebetween. In some embodiments, the replacement heart valve implant 216 may additionally and/or optionally include a sealing member disposed around and/or on an outer surface of the tubular anchor member 222. In at least some embodiments, the sealing member may be similar in form, construction, and/or function to the seal member 146 discussed above. Other configurations are also contemplated.

A replacement heart valve system according to the disclosure includes the expandable docking station 130 configured for implantation within a native heart valve, the expandable docking station 130 including the plurality of sacrificial valve leaflets 150 disposed within the braided anchoring element 132 defining the lumen 144 extending through the braided anchoring element 132. A replacement heart valve system according to the disclosure includes the replacement heart valve implant 216 configured to be disposed within the lumen 144 of the expandable docking station 130, the replacement heart valve implant 216 including the plurality of valve leaflets 220 disposed within the tubular anchor member 222 defining the lumen extending through the tubular anchor member 222. A replacement heart valve system according to the disclosure may include the docking station delivery device 100, wherein the expandable docking station 130 is disposed within the lumen 112 of the docking station delivery device 100 and/or the delivery sheath 110 in the delivery configuration for delivery to a native heart valve. A replacement heart valve system according to the disclosure may include the replacement heart valve delivery device 200, wherein the replacement heart valve implant 216 is disposed within the lumen of the replacement heart valve delivery device 200 and/or the delivery sheath 212 in the delivery configuration for delivery to the expandable docking station 130 disposed within the native heart valve in the deployed configuration.

A method of deploying a replacement heart valve implant 216 within a native heart valve (e.g., aortic valve 16, mitral valve 18, etc.) may comprise advancing the docking station delivery device 100 having the expandable docking station 130 disposed within the lumen 112 of the docking station delivery device 100 and/or the delivery sheath 110 in the delivery configuration to the native heart valve, wherein the expandable docking station 130 includes the plurality of sacrificial valve leaflets 150 disposed within the braided anchoring element 132 defining the lumen 144 extending through the braided anchoring element 132. The method may comprise deploying the expandable docking station 130 from the docking station delivery device 100 and/or the lumen 112 of the delivery sheath 110 into the native heart valve and expanding the expandable docking station 130 to the deployed configuration. In some embodiments, deploying the expandable docking station 130 may include clamping native valve leaflets of the native heart valve between the upstream flange 140 and the downstream flange 142 of the braided anchoring element 132.

The method of deploying the replacement heart valve implant 216 may include advancing the replacement heart valve delivery device 200 having the replacement heart valve implant 216 disposed within the lumen of the replacement heart valve delivery device 200 and/or the delivery sheath 212 in the delivery configuration to the native heart valve and/or the expandable docking station 130. The method of deploying the replacement heart valve implant 216 may include deploying the replacement heart valve implant 216 from replacement heart valve delivery device 200 into and/or within the lumen 144 of the braided anchoring element 132 and expanding the replacement heart valve implant 216 and/or the tubular anchor member 222 to the deployed configuration. In some embodiments, deploying the replacement heart valve implant 216 within the lumen 144 of the braided anchoring element 132 and/or expandable docking station 130 compresses the plurality of sacrificial valve leaflets 150 between an outer surface of the tubular anchor member 222 and an interior surface of the braided anchoring element 132, thereby forming the fluid tight seal therebetween.

In some embodiments, the expandable docking station 130 and the replacement heart valve implant 216 may be delivered to the native heart valve using a single delivery device or delivery sheath, (e.g., the same delivery device and/or delivery sheath). In some embodiments, the implants and/or medical devices may be delivered and/or placed in a method involving multiple delivery devices and a single access point or multiple access points into the patient, depending on the approach used. Other configuration, arrangements, and/or methods are also contemplated.

The materials that can be used for the various components of the replacement heart valve system, the delivery sheath(s) 50, and/or the implants and/or medical devices 52, the docking station delivery device 100, the expandable docking station 130, the replacement heart valve delivery device 200, the replacement heart valve implant 216, etc. (and/or other systems or components disclosed herein) and the various elements thereof disclosed herein may include those commonly associated with medical devices. For simplicity purposes, the following discussion makes reference to the replacement heart valve system, the docking station delivery device 100, the expandable docking station 130, the replacement heart valve delivery device 200, the replacement heart valve implant 216, etc. However, this is not intended to limit the devices and methods described herein, as the discussion may be applied to other elements, members, components, or devices disclosed herein, such as, but not limited to, the delivery sheath(s) 110/212, the elongate shaft(s) 120/214, the braided anchoring element 132, the tubular anchor member 222, etc. and/or elements or components thereof.

In some embodiments, the replacement heart valve system, the docking station delivery device 100, the expandable docking station 130, the replacement heart valve delivery device 200, the replacement heart valve implant 216, etc., and/or components thereof may be made from a metal, metal alloy, polymer (some examples of which are disclosed below), a metal-polymer composite, ceramics, combinations thereof, and the like, or other suitable material. Some examples of suitable metals and metal alloys include stainless steel, such as 444V, 444L, and 314LV stainless steel; mild steel; nickel-titanium alloy such as linear-elastic and/or super-elastic nitinol; other nickel alloys such as nickel-chromium-molybdenum alloys (e.g., UNS: N06625 such as INCONEL^{®} 625, UNS: N06022 such as HASTELLOY^{®} C-22^{®}, UNS: N10276 such as HASTELLOY^{®} C276^{®}, other HASTELLOY^{®} alloys, and the like), nickel-copper alloys (e.g., UNS: N04400 such as MONEL^{®} 400, NICKEL VAC^{®} 400, NICORROS^{®} 400, and the like), nickel-cobalt-chromium-molybdenum alloys (e.g., UNS: R44035 such as MP35-N^{®} and the like), nickel-molybdenum alloys (e.g., UNS: N10665 such as HASTELLOY^{®} ALLOY B2^{®}), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R44003 such as ELGILOY^{®}, PHYNOX^{®}, and the like); platinum enriched stainless steel; titanium; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear than the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also be distinguished based on its composition), which may accept only about 0.2 to 0.44 percent strain before plastically deforming.

In some embodiments, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some embodiments, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some embodiments, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties.

In some embodiments, the linear elastic and/or non-super-elastic nickel-titanium alloy may be in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Other suitable materials may include ULTANIUM^{™} (available from Neo-Metrics) and GUM METAL^{™} (available from Toyota). In some other embodiments, a superelastic alloy, for example a superelastic nitinol can be used to achieve desired properties.

In at least some embodiments, portions or all of the replacement heart valve system, the docking station delivery device 100, the expandable docking station 130, the replacement heart valve delivery device 200, the replacement heart valve implant 216, etc., and/or components thereof, may also be doped with, made of, or otherwise include a radiopaque material. Radiopaque materials are understood to be materials capable of producing a relatively bright image on a fluoroscopy screen or another imaging technique during a medical procedure. This relatively bright image aids a user in determining the location of the replacement heart valve system, the docking station delivery device 100, the expandable docking station 130, the replacement heart valve delivery device 200, the replacement heart valve implant 216, etc. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of the replacement heart valve system, the docking station delivery device 100, the expandable docking station 130, the replacement heart valve delivery device 200, the replacement heart valve implant 216, etc. to achieve the same result.

In some embodiments, a degree of Magnetic Resonance Imaging (MRI) compatibility is imparted into the replacement heart valve system, the docking station delivery device 100, the expandable docking station 130, the replacement heart valve delivery device 200, the replacement heart valve implant 216, etc. For example, the replacement heart valve system, the docking station delivery device 100, the expandable docking station 130, the replacement heart valve delivery device 200, the replacement heart valve implant 216, etc., and/or components or portions thereof, may be made of a material that does not substantially distort the image and create substantial artifacts (e.g., gaps in the image). Certain ferromagnetic materials, for example, may not be suitable because they may create artifacts in an MRI image. The replacement heart valve system, the docking station delivery device 100, the expandable docking station 130, the replacement heart valve delivery device 200, the replacement heart valve implant 216, etc., or portions thereof, may also be made from a material that the MRI machine can image. Some materials that exhibit these characteristics include, for example, tungsten, cobalt-chromium-molybdenum alloys (e.g., UNS: R44003 such as ELGILOY^{®}, PHYNOX^{®}, and the like), nickel-cobalt-chromium-molybdenum alloys (e.g., UNS: R44035 such as MP35-N^{®} and the like), nitinol, and the like, and others.

In some embodiments, the replacement heart valve system, the docking station delivery device 100, the expandable docking station 130, the plurality of sacrificial valve leaflets 150, the replacement heart valve delivery device 200, the replacement heart valve implant 216, the plurality of valve leaflets 220, etc., and/or portions thereof, may be made from or include a polymer or other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN^{®} available from DuPont), polyether block ester, polyurethane (for example, Polyurethane 85A), polypropylene (PP), polyvinylchloride (PVC), polyether-ester (for example, ARNITEL^{®} available from DSM Engineering Plastics), ether or ester based copolymers (for example, butylene/poly(alkylene ether) phthalate and/or other polyester elastomers such as HYTREL^{®} available from DuPont), polyamide (for example, DURETHAN^{®} available from Bayer or CRISTAMID^{®} available from Elf Atochem), elastomeric polyamides, block polyamide/ethers, polyether block amide (PEBA, for example available under the trade name PEBAX^{®}), ethylene vinyl acetate copolymers (EVA), silicones, polyethylene (PE), Marlex high-density polyethylene, Marlex low-density polyethylene, linear low density polyethylene (for example REXELL^{®}), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR^{®}), polysulfone, nylon, nylon-12 (such as GRILAMID^{®} available from EMS American Grilon), perfluoro(propyl vinyl ether) (PFA), ethylene vinyl alcohol, polyolefin, polystyrene, epoxy, polyvinylidene chloride (PVdC), poly(styrene-b-isobutylene-b-styrene) (for example, SIBS and/or SIBS 50A), polycarbonates, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

In some embodiments, the replacement heart valve system, the docking station delivery device 100, the expandable docking station 130, the replacement heart valve delivery device 200, the replacement heart valve implant 216, etc. may include and/or be formed from a textile material. Some examples of suitable textile materials may include synthetic yarns that may be flat, shaped, twisted, textured, pre-shrunk or un-shrunk. Synthetic biocompatible yarns suitable for use in the present invention include, but are not limited to, polyesters, including polyethylene terephthalate (PET) polyesters, polypropylenes, polyethylenes, polyurethanes, polyolefins, polyvinyls, polymethylacetates, polyamides, naphthalene dicarboxylene derivatives, natural silk, and polytetrafluoroethylenes. Moreover, at least one of the synthetic yarns may be a metallic yarn or a glass or ceramic yarn or fiber. Useful metallic yarns include those yarns made from or containing stainless steel, platinum, gold, titanium, tantalum or a Ni-Co-Cr-based alloy. The yarns may further include carbon, glass or ceramic fibers. Desirably, the yarns are made from thermoplastic materials including, but not limited to, polyesters, polypropylenes, polyethylenes, polyurethanes, polynaphthalenes, polytetrafluoroethylenes, and the like. The yarns may be of the multifilament, monofilament, or spun-types. The type and denier of the yarn chosen may be selected in a manner which forms a biocompatible and implantable prosthesis and, more particularly, a vascular structure having desirable properties.

In some embodiments, the replacement heart valve system, the docking station delivery device 100, the expandable docking station 130, the replacement heart valve delivery device 200, the replacement heart valve implant 216, etc. may include and/or be treated with a suitable therapeutic agent. Some examples of suitable therapeutic agents may include anti-thrombogenic agents (such as heparin, heparin derivatives, urokinase, and PPack (dextrophenylalanine proline arginine chloromethylketone)); anti-proliferative agents (such as enoxaparin, angiopeptin, monoclonal antibodies capable of blocking smooth muscle cell proliferation, hirudin, and acetylsalicylic acid); anti-inflammatory agents (such as dexamethasone, prednisolone, corticosterone, budesonide, estrogen, sulfasalazine, and mesalamine); antineoplastic/antiproliferative/anti-mitotic agents (such as paclitaxel, 5-fluorouracil, cisplatin, vinblastine, vincristine, epothilones, endostatin, angiostatin and thymidine kinase inhibitors); anesthetic agents (such as lidocaine, bupivacaine, and ropivacaine); anti-coagulants (such as D-Phe-Pro-Arg chloromethyl keton, an RGD peptide-containing compound, heparin, anti-thrombin compounds, platelet receptor antagonists, anti-thrombin antibodies, anti-platelet receptor antibodies, aspirin, prostaglandin inhibitors, platelet inhibitors, and tick antiplatelet peptides); vascular cell growth promoters (such as growth factor inhibitors, growth factor receptor antagonists, transcriptional activators, and translational promoters); vascular cell growth inhibitors (such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin); cholesterol-lowering agents; vasodilating agents; and agents which interfere with endogenous vascoactive mechanisms.

It should be understood that this disclosure is, in many respects, only illustrative. Changes may be made in details, particularly in matters of shape, size, and arrangement of steps without exceeding the scope of the invention. This may include, to the extent that it is appropriate, the use of any of the features of one example embodiment being used in other embodiments. The invention's scope is, of course, defined in the language in which the appended claims are expressed.

## Claims

1. A replacement heart valve system, comprising:
an expandable docking station (130) configured for implantation within a native heart valve (12, 14, 16, 18), the expandable docking station (130) including a plurality of sacrificial valve leaflets (150) disposed within an anchoring element (132) defining a lumen (144) extending through the braided anchoring element (132); and
a replacement heart valve implant (216) configured to be disposed within the lumen (144) of the expandable docking station (130), the replacement heart valve implant (216) including a plurality of valve leaflets (220) disposed within a tubular anchor member (222) defining a lumen extending through the tubular anchor member (222).

2. The replacement heart valve system of claim 1, wherein the plurality of sacrificial valve leaflets (150) is configured to move between an open configuration permitting antegrade fluid flow through the expandable docking station (130) and a closed configuration preventing retrograde fluid flow through the expandable docking station (130).

3. The replacement heart valve system of any of claims 1-2, wherein the plurality of valve leaflets (150) is configured to move between an open configuration permitting antegrade fluid flow through the replacement heart valve implant (216) and a closed configuration preventing retrograde fluid flow through the replacement heart valve implant (216).

4. The replacement heart valve system of any of claims 1-3, wherein the anchoring element (132) is configured to shift between an elongated delivery configuration and a shortened, radially expanded deployed configuration.

5. The replacement heart valve system of claim 4, wherein the anchoring element (132) is configured to clamp native valve leaflets of the native heart valve (12, 14, 16, 18) in the deployed configuration.

6. The replacement heart valve system of claim 5, wherein the anchoring element (132) comprises an upstream flange (140), a downstream flange (142), and a central portion (136) disposed between the upstream flange (140) and the downstream flange (142).

7. The replacement heart valve system of claim 6, wherein the upstream flange (140) extends radially outward of the central portion (136) in the deployed configuration.

8. The replacement heart valve system of any of claims 6-7, wherein the downstream flange (142) extends radially outward of the central portion (136) in the deployed configuration.

9. The replacement heart valve system of any of claims 6-8, wherein the expandable docking station (130) includes a seal member (146) disposed on at least a portion of an outer surface of the anchoring element (132).

10. The replacement heart valve system of claim 9, wherein the seal member (146) is disposed on the central portion (136) of the anchoring element (132).

11. The replacement heart valve system of any of claims 1-10, wherein the tubular anchor member (222) is configured to shift between an elongated delivery configuration and a shortened, radially expanded deployed configuration.

12. The replacement heart valve system of claim 11, wherein the tubular anchor member (222) is configured to engage the anchoring element (132) in the deployed configuration of the tubular anchor member (222).

13. The replacement heart valve system of claim 12, wherein when the tubular anchor member (222) is in the deployed configuration within the anchoring element (132), the plurality of sacrificial valve leaflets (150) is compressed between the tubular anchor member (222) and the anchoring element (132) thereby forming a fluid tight seal therebetween.

14. The replacement heart valve system of any of claims 1-13, wherein the expandable docking station (130) includes a leaflet subframe (160) disposed within the lumen of the anchoring element (132).

15. The replacement heart valve system of any of claims 1-14, further comprising:
a docking station delivery device (100), wherein the expandable docking station (130) is disposed within a lumen (112) of the docking station delivery device (100) in the delivery configuration for delivery to the native heart valve (12, 14, 16, 18); and
a replacement heart valve delivery device, wherein the replacement heart valve implant (216) is disposed within a lumen of the replacement heart valve delivery device (200) in the delivery configuration for delivery to the expandable docking station (130) disposed within the native heart valve (12, 14, 16, 18).

## Patentansprüche

1. Herzklappenersatzsystem, umfassend:
eine expandierbare Docking-Station (130), die für die Implantierung in eine native Herzklappe (12, 14, 16, 18) ausgestaltet ist, wobei die expandierbare Docking-Station (130) eine Vielzahl von Opferklappensegeln (150) aufweist, die in einem Verankerungselement (132) angeordnet sind, das ein Lumen (144) definiert, das sich durch das geflochtene Verankerungselement (132) erstreckt, und
ein Herzklappenersatzimplantat (216), das dazu ausgestaltet ist, in dem Lumen (144) der expandierbaren Docking-Station (130) angeordnet zu werden, wobei das Herzklappenersatzimplantat (216) eine Vielzahl von Klappensegeln (220) aufweist, die in einem röhrenförmigen Ankerglied (222) angeordnet sind, das ein Lumen definiert, das sich durch das röhrenförmige Ankerglied (222) erstreckt.

2. Herzklappenersatzsystem nach Anspruch 1, wobei die Vielzahl von Opferklappensegeln (150) dazu ausgestaltet sind, sich zwischen einer offenen Konfiguration, die antegrade Fluidströmung durch die expandierbare Docking-Station (130) gestattet, und einer geschlossenen Konfiguration, die eine retrograde Fluidströmung durch die expandierbare Docking-Station (130) verhindert, zu bewegen.

3. Herzklappenersatzsystem nach einem der Ansprüche 1-2, wobei die Vielzahl von Klappensegeln (150) dazu ausgestaltet sind, sich zwischen einer offenen Konfiguration, die antegrade Fluidströmung durch das Herzklappenersatzimplantat (216) gestattet, und einer geschlossenen Konfiguration, die eine retrograde Fluidströmung durch das Herzklappenersatzimplantat (216) verhindert, zu bewegen.

4. Herzklappenersatzsystem nach einem der Ansprüche 1-3, wobei das Verankerungselement (132) dazu ausgestaltet ist, zwischen einer länglichen Zuführkonfiguration und einer verkürzten, radial expandierten ausgebrachten Konfiguration zu wechseln.

5. Herzklappenersatzsystem nach Anspruch 4, wobei das Verankerungselement (132) dazu ausgestaltet ist, native Klappensegel der nativen Herzklappe (12, 14, 16, 18) in der ausgebrachten Konfiguration zu klemmen.

6. Herzklappenersatzsystem nach Anspruch 5, wobei das Verankerungselement (132) einen vorgeschalteten Flansch (140), einen nachgeschalteten Flansch (142) und einen mittleren Abschnitt (136) umfasst, der zwischen dem vorgeschalteten Flansch (140) und dem nachgeschalteten Flansch (142) angeordnet ist.

7. Herzklappenersatzsystem nach Anspruch 6, wobei sich der vorgeschaltete Flansch (140) in der ausgebrachten Konfiguration von dem mittleren Abschnitt (136) radial nach außen erstreckt.

8. Herzklappenersatzsystem nach einem der Ansprüche 6-7, wobei sich der nachgeschaltete Flansch (142) in der ausgebrachten Konfiguration von dem mittleren Abschnitt (136) radial nach außen erstreckt.

9. Herzklappenersatzsystem nach einem der Ansprüche 6-8, wobei die expandierbare Docking-Station (130) ein Dichtungsglied (146) aufweist, das an mindestens einem Teil einer Außenfläche des Verankerungselements (132) angeordnet ist.

10. Herzklappenersatzsystem nach Anspruch 9, wobei das Dichtungsglied (146) an dem mittleren Abschnitt (136) des Verankerungselements (132) angeordnet ist.

11. Herzklappenersatzsystem nach einem der Ansprüche 1-10, wobei das röhrenförmige Ankerglied (222) dazu ausgestaltet ist, zwischen einer länglichen Zuführkonfiguration und einer verkürzten, radial expandierten ausgebrachten Konfiguration zu wechseln.

12. Herzklappenersatzsystem nach Anspruch 11, wobei das röhrenförmige Ankerglied (222) dazu ausgestaltet ist, das Verankerungselement (132) in der ausgebrachten Konfiguration des röhrenförmigen Ankerglieds (222) in Eingriff zu nehmen.

13. Herzklappenersatzsystem nach Anspruch 12, wobei, wenn das röhrenförmige Ankerglied (222) in der ausgebrachten Konfiguration in dem Verankerungselement (132) ist, die Vielzahl von Opferklappensegeln (150) zwischen dem röhrenförmigen Ankerglied (222) und dem Verankerungselement (132) zusammengedrückt werden, wodurch eine fluiddichte Abdichtung dazwischen gebildet wird.

14. Herzklappenersatzsystem nach einem der Ansprüche 1-13, wobei die expandierbare Docking-Station (130) einen Segel-Unterrahmen (160) aufweist, der in dem Lumen des Verankerungselements (132) angeordnet ist.

15. Herzklappenersatzsystem nach einem der Ansprüche 1-14, ferner umfassend:
eine Vorrichtung (100) zur Zuführung der Docking-Station, wobei die expandierbare Docking-Station (130) in einem Lumen (112) der Vorrichtung (100) zur Zuführung der Docking-Station in der Zuführungskonfiguration zur Zuführung zu der nativen Herzklappe (12, 14, 16, 18) angeordnet ist, und
eine Herzklappenersatzzufuhrvorrichtung, wobei das Herzklappenersatzimplantat (216) in einem Lumen der Herzklappenersatzzufuhrvorrichtung (200) in der Zuführungskonfiguration zur Zuführung zu der expandierbaren Docking-Station (130) angeordnet ist, die in der nativen Herzklappe (12, 14, 16, 18) angeordnet ist.

## Revendications

1. Système de valvule cardiaque de remplacement, comprenant :
une station d'attache extensible (130) conçue pour être implantée à l'intérieur d'une valvule cardiaque native (12, 14, 16, 18), la station d'attache extensible (130) comportant une pluralité de feuillets valvulaires sacrificiels (150) disposés à l'intérieur d'un élément d'ancrage (132) définissant une lumière (144) s'étendant à travers l'élément d'ancrage tressé (132) ; et
un implant de valvule cardiaque de remplacement (216) conçu pour être disposé à l'intérieur de la lumière (144) de la station d'attache extensible (130), l'implant de valvule cardiaque de remplacement (216) comportant une pluralité de feuillets valvulaires (220) disposés à l'intérieur d'un élément d'ancrage tubulaire (222) définissant une lumière s'étendant à travers l'élément d'ancrage tubulaire (222).

2. Système de valvule cardiaque de remplacement selon la revendication 1, la pluralité de feuillets valvulaires sacrificiels (150) étant conçue pour se déplacer entre une configuration ouverte permettant un écoulement de fluide antérograde à travers la station d'attache extensible (130) et une configuration fermée empêchant un écoulement de fluide rétrograde à travers la station d'attache extensible (130).

3. Système de valvule cardiaque de remplacement selon l'une quelconque des revendications 1 et 2, la pluralité de feuillets valvulaires (150) étant conçue pour se déplacer entre une configuration ouverte permettant un écoulement de fluide antérograde à travers l'implant de valvule cardiaque de remplacement (216) et une configuration fermée empêchant un écoulement de fluide rétrograde à travers l'implant de valvule cardiaque de remplacement (216).

4. Système de valvule cardiaque de remplacement selon l'une quelconque des revendications 1 à 3, l'élément d'ancrage (132) étant conçu pour basculer entre une configuration de mise en place allongée et une configuration déployée raccourcie, radialement étendue.

5. Système de valvule cardiaque de remplacement selon la revendication 4, l'élément d'ancrage (132) étant conçu pour serrer des feuillets valvulaires natifs de la valvule cardiaque native (12, 14, 16, 18) dans la configuration déployée.

6. Système de valvule cardiaque de remplacement selon la revendication 5, l'élément d'ancrage (132) comprenant une bride amont (140), une bride aval (142) et une partie centrale (136) disposée entre la bride amont (140) et la bride aval (142).

7. Système de valvule cardiaque de remplacement selon la revendication 6, la bride amont (140) s'étendant radialement vers l'extérieur de la partie centrale (136) dans la configuration déployée.

8. Système de valvule cardiaque de remplacement selon l'une quelconque des revendications 6 et 7, la bride aval (142) s'étendant radialement vers l'extérieur de la partie centrale (136) dans la configuration déployée.

9. Système de valvule cardiaque de remplacement selon l'une quelconque des revendications 6 à 8, la station d'attache extensible (130) comportant un élément d'étanchéité (146) disposé sur au moins une partie d'une surface extérieure de l'élément d'ancrage (132).

10. Système de valvule cardiaque de remplacement selon la revendication 9, l'élément d'étanchéité (146) étant disposé sur la partie centrale (136) de l'élément d'ancrage (132).

11. Système de valvule cardiaque de remplacement selon l'une quelconque des revendications 1 à 10, l'élément d'ancrage tubulaire (222) étant conçu pour basculer entre une configuration de mise en place allongée et une configuration déployée raccourcie, radialement étendue.

12. Système de valvule cardiaque de remplacement selon la revendication 11, l'élément d'ancrage tubulaire (222) étant conçu pour être en prise avec l'élément d'ancrage (132) dans la configuration déployée de l'élément d'ancrage tubulaire (222).

13. Système de valvule cardiaque de remplacement selon la revendication 12, lorsque l'élément d'ancrage tubulaire (222) est dans la configuration déployée à l'intérieur de l'élément d'ancrage (132), la pluralité de feuillets valvulaires sacrificiels (150) étant comprimée entre l'élément d'ancrage tubulaire (222) et l'élément d'ancrage (132), formant ainsi un joint étanche aux fluides entre eux.

14. Système de valvule cardiaque de remplacement selon l'une quelconque des revendications 1 à 13, la station d'attache extensible (130) comportant un sous-cadre de feuillets (160) disposé à l'intérieur de la lumière de l'élément d'ancrage (132).

15. Système de valvule cardiaque de remplacement selon l'une quelconque des revendications 1 à 14, comprenant en outre :
un dispositif de mise en place de station d'attache (100), la station d'attache extensible (130) étant disposée à l'intérieur d'une lumière (112) du dispositif de mise en place de station d'attache (100) dans la configuration de mise en place pour une mise en place sur la valvule cardiaque native (12, 14, 16, 18) ; et
un dispositif de mise en place de valvule cardiaque de remplacement, l'implant de valvule cardiaque de remplacement (216) étant disposé à l'intérieur d'une lumière du dispositif de mise en place de valvule cardiaque de remplacement (200) dans la configuration de mise en place pour une mise en place sur la station d'attache extensible (130) disposée à l'intérieur de la valvule cardiaque native (12, 14, 16, 18).
